# EUROPEAN PATENT SPECIFICATION

(11) **EP 3 868 359 B1**
(45) Date of publication and mention of the grant of the patent: **01.01.2025**
(21) Application number: 19874704.0
(22) Date of filing: 15.10.2019
(51) Int. Cl.: A61K 8/365, A61Q 19/00

(54) **COMPOSITION FOR STRENGTHENING SKIN BARRIER**
ZUSAMMENSETZUNG ZUR VERSTÄRKUNG DER HAUTBARRIERE
COMPOSITION POUR RENFORCER LA BARRIÈRE CUTANÉE

(30) Priority: 15.10.2018 KR 20180122682
(43) Date of publication of application: 25.08.2021
(73) Proprietor: Amorepacific Corporation, Seoul 04386 (KR)
(72) Inventor: CHOI, Eun Jeong, Giheung-gu Yongin-si Gyeonggi-do 17074 (KR); KIM, Hyoung June, Giheung-gu Yongin-si Gyeonggi-do 17074 (KR); LEE, Tae Ryong, Giheung-gu Yongin-si Gyeonggi-do 17074 (KR); CHOI, Hyunjung, Giheung-gu Yongin-si Gyeonggi-do 17074 (KR)
(74) Representative: Schön, Christoph
(86) International application number: PCT/KR2019/013516
(87) International publication number: WO 2020/080797

(56) References cited:
- WO-A1-2010/095706
- WO-A2-2006/055965
- KR-A- 20070 090 928
- KR-A- 20170 122 808
- KR-A- 20180 096 158
- KR-A- 20180 096 158
- US-A1- 2011 190 389
- US-A1- 2015 126 602
- US-A1- 2018 271 818

## Description

### [Technical Field]

This disclosure relates to a composition and a method of strengthening a skin barrier by applying it to the skin.

### [Background Art]

A skin performs various functions essential for the human body to survive. Barrier functions to maintain homeostasis inside the human body in response to environmental changes, sensory functions to recognize external changes, and body temperature control functions are among the most representative skin functions. Among the various functions of the skin, in particular, the barrier functions of the skin are mainly manifested by the stratum corneum at the outermost portion of the skin. Since the stratum corneum has been reported to affect functions, roles, structures, and the like of an inner living cell layer, that is, an epidermal layer or a dermal layer, as well as performs the simple barrier functions, its importance is constantly increasing. This stratum corneum is composed of dead keratinocytes and intercellular lipids, and plays a key function as a skin protective layer that protects the skin from external stimuli and prevents moisture from evaporating from the inside. In addition, the keratinocytes in the stratum corneum create a skin barrier through differentiation and keratinization processes.

There are various factors that cause aging in human skin. In particular, ultraviolet (UV) rays cause wrinkles, a decrease in elasticity, pigmentation, and a decrease in a skin moisture content due to damage on the skin barrier. When the skin surface moisture content decreases due to skin damage caused by the ultraviolet rays, the stratum corneum on the skin surface loses flexibility and thus makes the skin dry, eventually failing in properly functioning as a barrier. Therefore, in order to strengthen the skin barrier, it is very important to maintain moisturizing power of the skin.

There are two types of pores associated with water homeostasis present in the epidermis: Aquaporins (AQP) and Tight Junction (TJ). In addition, filagrin is a precursor protein of the natural moisturizing factor (NMF) responsible for moisturizing the skin, and it is known that its ability to promote filaggrin production plays an important role in the moisturizing action (J. Invest. 31, 1970).

On the other hand, various inflammatory dermatitises are caused by IgE-related immune mechanisms, and there are many reports that a delayed immune response caused by T-cell abnormalities is involved therein. In particular, around a skin area where atopic dermatitis has occurred, invasion of immune-related cells such as macrophages, Th lymphocytes, and mast cells is greatly increased. Patients with atopic dermatitis show a high IgE concentration in the blood, because the number of Th2 cells increases, these Th2 cells secrete Th2 cytokines such as IL-4, IL-13, and the like, which stimulate B lymphocytes, and IgE secretion is stimulated through stimulation of the B lymphocytes. In particular, in case of early atomic dermatitis, IL-4 and IL-13 play an important role (Donald Y.M. Leung et al., J Clin Invest. 2004, 113, 651-657).

In treatment and prevention of inflammation such as the atopic dermatitis, it is very important to make an inflammation-resolving factor actively involved. The inflammation-resolving factor in actively involved in inflammation resolution, and is naturally produced and secreted from immune cells (neutrophil, macrophage) in the tissues at the end of an inflammatory reaction. The inflammation-resolving factor has various forms such as lipids, proteins, and gas molecules, and particularly, an inflammation-resolving lipid factor (specialized pro-resolving lipid mediators, SPMs) among them have been actively studied in recent years. The inflammation-resolving lipid factor is a metabolite produced by metabolism of eicosapentaenoic acid (EPA) or docosahexaenoic acid (DHA), which is an omega-3 polyunsaturated fatty acid (PUFA), in cells.

Korean patent application KR-A- 2018/0096158 discloses the use of a composition comprising Maresin 1 for preventing or treating skin ageing, US patent application US-A-2018/271818 discloses the use of a composition comprising a similar 7S,14S-dihydroxy-docosahexaenoic acid (HDHA) to treat anti-inflammatory or resolution-stimulating activity such as make-up, skin moisturizers or specific topical creams such as sunburn and tanning ointments, and the International patent application WO-A-2006/055965 discloses 14-HDHA ethyl ester having anti-inflammatory activity in neutrophilic inflammation.

Up to now, there have been many reports on alleviating inflammation responses by the inflammation-resolving lipid factor, but uses of the inflammation-resolving lipid factor for strengthening the skin barrier and improving the moisturizing power have not yet been known. Accordingly, the inventors of the present disclosure discovered that the inflammation-resolving lipid factor has these aforementioned effects, while developing a natural skin barrier-strengthening agent, which is safe for the human body.

### [Disclosure]

### [Technical Problem]

An embodiment is to provide a (cosmetic) composition capable of improving skin moisturizing power by strengthening the damaged skin barrier, particularly the skin barrier damaged by ultraviolet rays.

### [Technical Solution]

According to an embodiment, a composition for strengthening a skin barrier, including a compound represented by Chemical Formula 1 as an active ingredient is provided.

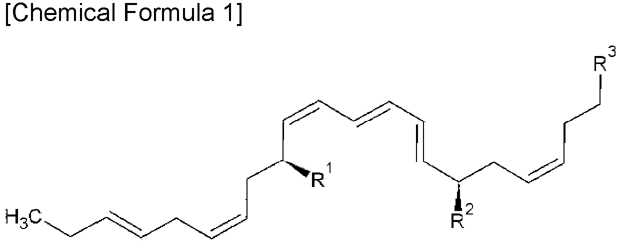

In Chemical Formula 1,
R¹ and R² are independently a hydroxy group and R³ is a carboxyl group.

The compound represented by Chemical Formula 1 is included in a concentration range of 0.01 pM to 100 µM.

The strengthening of the skin barrier may be characterized in that it improves skin moisturizing power by reducing moisture loss of the skin.

The strengthening of the skin barrier may be characterized by strengthening the skin barrier damaged by ultraviolet rays.

The composition may be a cosmetic composition.

According to another embodiment, a method of strengthening a skin barrier includes applying a composition including an effective amount of the compound represented by Chemical Formula 1 as an active ingredient, to the skin.

### [Advantageous Effects]

According to an embodiment, it is possible to strengthen the skin barrier by reducing a moisture loss of the skin and improving the skin barrier that is damaged by UV irradiation.

### [Description of the Drawings]

FIG. 1 is a graph measuring an effect on the expression of the keratinocyte differentiation marker gene (Keratin 1).
FIG. 2 is a graph measuring an effect on the expression of the keratinocyte differentiation marker gene (Keratin 10).
FIG. 3 is a graph measuring an effect on the expression of the DSC1 (Desmocollin-1) gene, which is an intercellular conjugate (desmosome) constituent factor.

### [Best Mode]

Hereinafter, embodiments of the present disclosure are described in detail so that those of ordinary skill in the art can easily implement the present disclosure. However, this disclosure may be embodied in many different forms.

In the present specification, improving the skin barrier function means strengthening the barrier of the stratum corneum on the outer shell of the skin. The stratum corneum, a primary barrier of the skin, may be easily damaged by the external environment, and in case of a dry skin, the skin barrier corneum does not properly function or has been damaged. In order to moisturize the dry skin, a moisturizer may be generally applied as a temporary solution but may not be a fundamental solution to the problem, and furthermore, the skin barrier damaged by ultraviolet rays may not be recovered by simply applying the moisturizer. Accordingly, the inventors of the present disclosure have confirmed that a compound represented by a specific chemical formula of the present disclosure may solve the fundamental problem and strengthen the skin barrier, and then completed the present disclosure.

In the present specification, it will be understood that when an element such as a layer, film, region, or substrate is referred to as being "on" another element, it may be directly on the other element or intervening elements may also be present. In contrast, when an element is referred to as being "directly on" another element, there are no intervening elements present.

In the present specification, when a definition is not otherwise provided, the term "combination" refers to mixing or copolymerization. In addition, "copolymerization" means block copolymerization or random copolymerization, and "copolymer" means block copolymer or random copolymer.

Hereinafter, a composition for strengthening a skin barrier according to an embodiment is described.

A composition for strengthening a skin barrier according to an embodiment includes a compound represented by Chemical Formula 1 as an active ingredient.

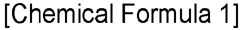

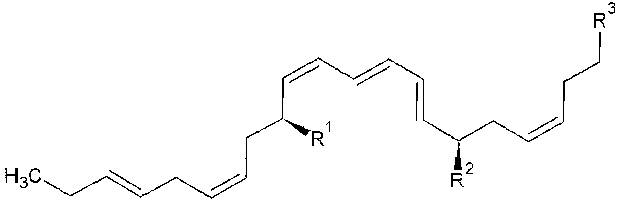

In Chemical Formula 1,
R1 and R2 are each independently a hydroxy group, and R3 is a carboxyl group.

The compound represented by Chemical Formula 1 is one of the inflammation-resolving lipid factors (specialized pro-resolving lipid mediators, SPMs), which strengthen damage of the skin barrier by ultraviolet rays and reduces moisture loss of the skin, thereby improving the moisturizing power of the skin. Specifically, when closely looking at the skin damaged by ultraviolet rays, an expression decrease of keratinocyte differentiation marker genes and desmosome factor genes in the skin is found, but the compound represented by Chemical Formula 1 may have an excellent effect in improving the skin barrier damaged by the ultraviolet rays by increasing the expression of the keratinocyte differentiation marker genes and the desmosome factor genes. In other words, the composition according to an embodiment includes the compound represented by Chemical Formula 1 as an active ingredient, and thus may have an excellent effect of strengthening the skin barrier damaged by the ultraviolet rays.

In addition, the compound represented by Chemical Formula 1 may strengthen the skin barrier by increasing the gene expression of the skin moisturizing factors, aquaporin and filaggrin, and finally providing moisturizing power to the skin. In other words, the composition according to an embodiment includes the compound represented by Chemical Formula 1 as an active ingredient, and thus may reduce the moisture loss of the skin and have an excellent effect in improving skin moisturizing power.

An embodiment provides a composition for strengthening a skin barrier including the compound represented by Chemical Formula 1 as an active ingredient, wherein the compound may include a pharmaceutically effective amount of the compound represented by Chemical Formula 1 alone or at least one pharmaceutically acceptable carrier, excipient, or diluent.

In the composition, the compound represented by Chemical Formula 1 is included in a concentration range of 0.01 pM to 100 µM. When the compound represented by Chemical Formula 1 is used as a cosmetic composition for strengthening a skin barrier, the compound represented by Chemical Formula 1 may be used at a concentration of greater than or equal to 0.01 pM, greater than or equal to 0.1 pM. The compound represented by Chemical Formula 1 may be used at a concentration of less than or equal to 100 µM, less than or equal to 10 µM. When the compound represented by Chemical Formula 1 is used at a concentration of less than 0.01 pM, the expression of the keratinocyte differentiation marker genes is insignificant, not obtaining the effect of strengthening the skin barrier function, but when the compound represented by Chemical Formula 1 is used at a concentration of greater than 100 µM, cytotoxicity harming the human body appears, which is not desirable.

In the above, "pharmaceutically effective amount" refers to an amount sufficient to allow the physiologically active ingredient to be administered to an animal or human to exhibit desired physiological or pharmacological activity. However, the effective amount of the pharmaceutical may vary according to the degrees of symptoms, ages, weights, health status, sexes, administration routes, and duration of treatment.

In addition, "pharmaceutically acceptable" refers to physiologically acceptable when administered to humans, and usually does not cause allergic reactions or similar reactions, such as gastrointestinal disorders or dizziness. Examples of the carrier, excipient, and diluent may include lactose, dextrose, sucrose, sorbitol, mannitol, xylitol, erythritol, maltitol, starch, acacia rubber, alginate, gelatin, calcium phosphate, calcium silicate, cellulose, methyl cellulose, polyvinylpyrrolidone, water, methylhydroxybenzoate, propylhydroxybenzoate, talc, magnesium stearate, and mineral oils. In addition, it may further include fillers, anti-coagulants, lubricants, wetting agents, fragrances, emulsifiers, and antiseptics.

For example, the composition may be a cosmetic composition.

In the present specification "cosmetic" may refer to any material that may have a medical function in addition to the cosmetic function.

The formulation of the cosmetic composition may be appropriately selected as desired.

For example, the cosmetic composition may be formulated into formulations such as solutions, suspension liquids, emulsions, pastes, gels, creams, lotions, powders, soaps, surfactant-containing cleansings, oils, powder foundations, emulsion foundations, wax foundations, and sprays. More specifically, it may be formulated into cosmetic compositions such as detergents, tonics, hair dressings, nourishing lotions, essences, serums, treatments, conditioners, shampoos, lotions, wools, hair dyes, and the like, and may be formulated into basic cosmetics such as an oil-in-water (O/W) type, a water-in-oil (W/O), and the like. For example, the composition may have a formulation selected from a skin lotion, a skin toner, an astringent, lotion, a milk lotion, a moisture lotion, a nutrition lotion, a massage cream, a nutrition cream, a moisture cream, a hand cream, an ointment, a foundation, an essence, a nutrition essence, a pack, a soap, a cleansing foam, a cleansing lotion, a cleansing cream, a body lotion, a body cleanser, a lotion, an ointment, a gel, a cream, a patch, and a spray. In addition, in the composition, in addition to the above-mentioned essential components in each formulation, other components may be appropriately selected and formulated without difficulty by a person of ordinary skill in the art according to types or use purposes of other external preparations. For example, ultraviolet blocking agents, hair conditioning agents, fragrances, and the like may be further included.

The cosmetic composition may include a cosmetically acceptable medium or base. These are all formulations suitable for topical applications. The cosmetic composition may be provided in the form of emulsions obtained by dispersing an oil phase in an aqueous phase, suspensions, microemulsions, microcapsules, microgranules, or ion-type (liposome) and/or non-ionized vesicle dispersing agents, or in the form of creams, skins, lotions, powders, ointments, sprays, or conceal sticks. These compositions may be prepared according to conventional methods in the art.

When the formulation of the present disclosure is a solution or emulsion, a solvent, a solubilizer, or an emulsifier may be used as carrier components. For example, water, ethanol, isopropanol, ethyl carbonate, ethyl acetate, benzyl alcohol, benzyl benzoate, propylene glycol, 1,3-butylglycol oil, glycerol aliphatic ester, polyethylene glycol, or fatty acid ester of sorbitan may be used.

If the formulation of the present disclosure is a suspension, the carrier component may be a diluent of a liquid such as water, ethanol, or propylene glycol, a suspending agent such as ethoxylated isostearyl alcohol, polyoxyethylene sorbitol ester, and polyoxyethylene sorbitan ester, microcrystalline cellulose, aluminum metahydroxide, bentonite, agar, tragacanth, and the like.

If the formulation of the present disclosure is pastes, creams, or gels, and the carrier component may be animal oil, vegetable oil, wax, paraffin, starch, tragacanth, cellulose derivatives, polyethylene glycol, silicone, bentonite, silica, talc, or zinc oxide.

If the formulation of the present disclosure is powders or sprays, the carrier component may be lactose, talc, silica, aluminum hydroxide, calcium silicate, or polyamide powders. Particularly, in the case of sprays, a propellant such as a chlorofluorohydrocarbon, propane/butane, or dimethyl ether may be additionally included.

In an embodiment of the present disclosure, the cosmetic composition may include thickeners. The thickeners included in the cosmetic composition of the present disclosure may be methyl cellulose, carboxyl methyl cellulose, carboxyl methyl hydroxy guanine, hydroxy methyl cellulose, hydroxyethyl cellulose, a carboxyl vinyl polymer, polyquaternium, cetearyl alcohol, stearic acid, and carrageenan, preferably one or more of carboxyl methyl cellulose, a carboxyl vinyl polymer, and polyquaternium may be used, and more preferably a carboxyl vinyl polymer may be used.

In an embodiment of the present disclosure, the cosmetic composition may include a variety of suitable bases and additives as needed, and the types and amounts of these components may be easily selected by the inventor. If necessary, it may include an acceptable additive, and may further include, for example, conventional ingredients such as antiseptics, pigments, additives, and the like.

The antiseptics may specifically be phenoxyethanol or 1,2-hexanediol, and the fragrances may be artificial fragrances.

In an embodiment of the present disclosure, the cosmetic composition may include a composition selected from a water-soluble vitamin, an oil-soluble vitamin, a polymeric peptide, a polymeric polysaccharide, a sphingolipid, and a seaweed extract. Other ingredients that may be added include fats and oils, humectants, emollients, surfactants, organic and inorganic pigments, organic powders, ultraviolet absorbers, antiseptics, fungicides, antioxidants, plant extracts, pH adjusters, alcohols, pigments, fragrances, blood circulation accelerators, coolants, anhidrotics, purified water, and the like.

In addition, the compounding components which may be added other than these. Moreover, any component may be blended in the range which does not damage the purpose and effect of the invention.

Furthermore, the cosmetic composition according to an embodiment may be used not only as a pharmaceutical composition as described above, but also as a dietary supplement. For example, it may be easily used as main ingredients, auxiliary ingredients, food ingredients, food additives, functional foods, or beverages.

The "food" means a natural or processed product including one or more nutrients, and preferably means that it is ready to be eaten directly after a certain amount of processing. It includes all foods, food additives, functional foods, and beverages.

The foods to which the food composition can be added may include, for example, various foods, beverages, gums, teas, vitamin composites, and functional foods. In addition, the foods may include special nutritional products (e.g., formulas, baby food, etc.), processed meat products, fish products, tofu, jellies, noodles (e.g. ramen noodles, etc.), breads, dietary supplements, seasoned foods (e.g., soy sauce, soybean paste, red pepper paste, mixed soy sauce, etc.), sauces, sweets (e.g. snacks), candy, chocolate, gum, ice cream, dairy products (e.g. fermented milk, cheese, etc.), other processed foods, kimchi, pickles (various kimchi, pickles, etc.), beverages (e.g., fruit beverages, vegetable beverages, soy milk, fermented beverages, etc.), and natural seasonings (e.g., ramen soup, etc.). The foods, beverages, or food additives may be prepared by conventional manufacturing methods.

In addition, "functional foods" or "health functional foods" refers to a food group that has added values to foods by using physical, biochemical, or biotechnological techniques to act and express functions of foods for specific purposes, or foods that are processed and designed to fully express the body's regulatory functions, such as defense rhythm control of food compositions, disease prevention, and recovery of living bodies. It may specifically be a health functional food. The functional food may include acceptable food auxiliary additives, and may further include suitable carriers, excipients, and diluents commonly used in the manufacture of functional foods.

The types of dietary supplements may be in a form of powders, granules, tablets, capsules, or beverages.

According to another embodiment, a method of strengthening a skin barrier includes applying a composition including an effective amount of the compound represented by Chemical Formula 1 as an active ingredient, to the skin.

Advantages and features of the present disclosure and methods for achieving them will be apparent with reference to the examples described in detail below. The present disclosure is described in detail with reference to examples. However, these examples are specifically provided for describing the present disclosure.

### [Mode for Invention]

### (Examples)

### Experimental Example: Confirmation of Expression Level of Keratinocyte Differentiation Marker (KRT1, KRT10) and Desmosome Constituent Factor (DSC1)

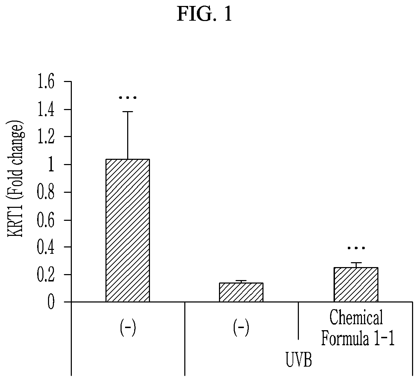

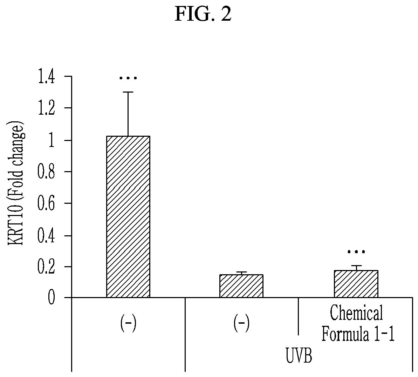

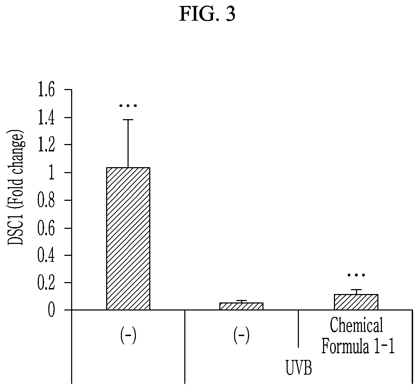

Normal human epidermal keratinocytes (NHEK) were cultured in a 6-well plate incubator. 24 hours later, the normal human epidermal keratinocytes (NHEK) were cultured by replacing it with phosphate-buffered saline (PBS) and irradiating it with UVB (25 mJ/cm²) and adding 10 nM of a compound represented by Chemical Formula 1-1 (Cayman Chemical) to the NHEK culture medium. On the fourth day of the culture, cells were harvested to separate RNA and synthesize cDNA through RT-PCR (a reverse transcriptional polymerase chain reaction), and the synthesized cDNA was used to perform Taqman real-time PCR and measure gene expression levels of a keratinocyte differentiation markers, KRT1 and KRT10, and a desmosome constituent factor, DSC1, and the results are shown in FIGS. 1 to 3. Referring to FIGS. 1 to 3, the gene expression levels of KRT1 (keratin 1), KRT10 (keratin 10), and DSC1 decreased after the ultraviolet irradiation, but the gene expression levels of KRT1 (keratin 1), KRT10 (keratin 10), and DSC1 increased again after treated with the compound represented by Chemical Formula 1-1, and accordingly, the compound represented by Chemical Formula 1-1 improved a skin barrier damaged by the ultraviolet rays.

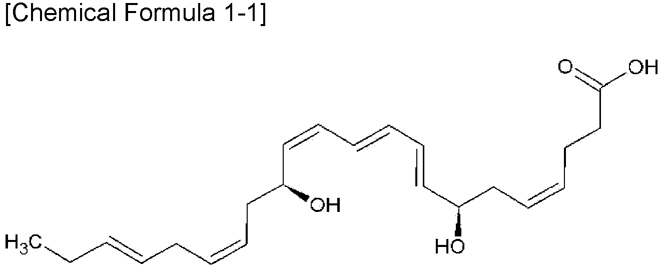

Although the preferred embodiments of the present disclosure have been described in detail, various modifications and improvements by those skilled in the art using the basic concept of the present disclosure defined in the following claims are also within the scope of the invention.

## Claims

1. Non-therapeutic use of a composition comprising a compound represented by Chemical Formula 1 as an active ingredient in a concentration range of 0.01 µM to 100 µM for strengthening a skin barrier: 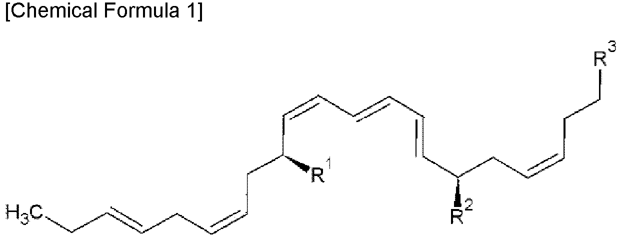
wherein, in Chemical Formula 1,
R¹ and R² are each independently a hydroxy group, and
R³ is a carboxyl group.

2. The non-therapeutic use of claim 1, wherein the composition improves skin moisturizing power by reducing moisture loss of the skin.

3. The non-therapeutic use of claim 1 or 2, wherein the composition is a cosmetic composition.

4. The non-therapeutic use of claims 1 to 3, wherein the composition is for strengthening the skin barrier damaged by ultraviolet rays.

## Patentansprüche

1. Nicht-therapeutische Verwendung einer Zusammensetzung, die eine durch die chemische Formel 1 dargestellte Verbindung als einen wirksamen Bestandteil in einem Konzentrationsbereich von 0,01 µM bis 100 µM umfasst, zur Stärkung der Hautbarriere: 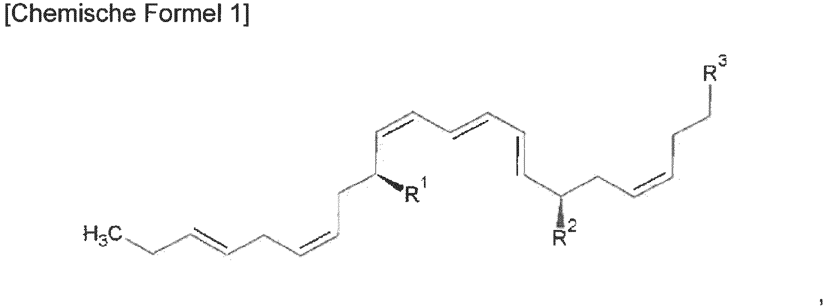
wobei in der chemischen Formel 1
R¹ und R² jeweils unabhängig für eine Hydroxygruppe stehen, und
R³ für eine Carboxylgruppe steht.

2. Nicht-therapeutische Verwendung gemäß Anspruch 1, wobei die Zusammensetzung das Feuchthaltevermögen der Haut durch Verringern des Feuchtigkeitsverlusts der Haut verbessert.

3. Nicht-therapeutische Verwendung gemäß Anspruch 1 oder Anspruch 2, wobei die Zusammensetzung eine kosmetische Zusammensetzung ist.

4. Nicht-therapeutische Verwendung gemäß den Ansprüchen 1 bis 3, wobei die Zusammensetzung zur Stärkung der durch Ultraviolettstrahlen geschädigten Hautbarriere vorgesehen ist.

## Revendications

1. Usage non thérapeutique d'une composition comprenant un composé représenté par la formule chimique 1 comme ingrédient actif dans une plage de concentration de 0,01 µM à 100 µM pour renforcer une barrière de peau : 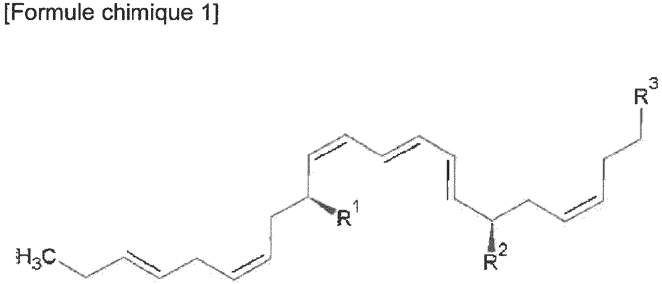
dans laquelle, dans la formule chimique 1,
R¹ et R² sont chacun indépendamment un groupe hydroxy et
R³ est un groupe carboxyle.

2. Usage non thérapeutique selon la revendication 1, dans lequel la composition améliore le pouvoir d'hydratation de la peau en réduisant la perte d'humidité de la peau.

3. Usage non thérapeutique selon la revendication 1 ou 2, dans lequel la composition est une composition cosmétique.

4. Usage non thérapeutique selon les revendications 1 à 3, dans lequel la composition est pour le renforcement de la barrière de peau endommagée par les rayons ultraviolets.
